# DEMANDE DE BREVET EUROPEEN

(11) **EP 1 498 113 A1**
(43) Date de publication de la demande: **19.01.2005**
(21) Numéro de dépôt: 04291736.9
(22) Date de dépôt: 08.07.2004
(51) Int. Cl.: A61K 7/48

(54) **Utilisation de béta-endorphine et/ou d'agents exercant une activité béta-endorphine-like en cosmétique et dermatologie**

(30) Priorité: 17.07.2003 FR 0308727
(71) Demandeur: L'OREAL, 75008 Paris (FR)
(72) Inventeur: Catroux, Philippe, 94130 Nogent sur Marne (FR); Dalko, Maria, 91190 Gif S/Yvette (FR)
(74) Mandataire: Allab, Myriam

(57) **Abrégé**

L'invention a pour objet l'utilisation d'au moins un composé choisi parmi la β-endorphine, et les agents mimétiques de la β-endorphine sur les kératinocytes de la peau, dans une composition cosmétique pour améliorer la fonction barrière de la peau, renforcer sa résistance aux stress et notamment à la pollution. Elle concerne également un procédé de traitement cosmétique pour améliorer la fonction barrière et/ou l'hydratation de la peau et/ou des muqueuses.

## Description

L'invention a pour objet l'utilisation d'agents exerçant une activité beta-endorphine like en cosmétique et dermatologie. L'invention se rapporte également aux utilisations des agents pré-cités dans une composition ou pour la préparation d'une composition pour la peau et/ou les cheveux, ainsi qu'à un procédé de traitement cosmétique de la peau et/ou des cheveux.

La peau constitue une barrière contre les agressions extérieures, notamment chimiques, mécaniques ou infectieuses, et à ce titre un certain nombre de réactions de défense contre les facteurs environnementaux (climat, rayons ultraviolets, tabac, ...) et/ou les xénobiotiques, comme par exemple les micro-organismes, se produisent à son niveau.
La peau est constituée de deux compartiments, l'un superficiel, l'épiderme, et un plus profond, le derme, qui interagissent. L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau, notamment le rôle de protection de l'organisme des agressions extérieures appelé "fonction barrière".
L'épiderme est conventionnellement divisé en une couche basale de kératinocytes constituant la couche germinative de l'épiderme, une couche dite épineuse constituée de plusieurs couches de cellules polyédriques disposées sur les couches germinatives, une à trois couches dites granuleuses constituées de cellules aplaties contenant des inclusions cytoplasmiques distinctes, les grains de kératohyaline et enfin la couche cornée (ou *stratum corneum*), constituée d'un ensemble de couches de kératinocytes au stade terminal de leur différenciation appelés cornéocytes.
Le derme fournit à l'épiderme un support solide. C'est également son élément nourricier. Il est principalement constitué de fibroblastes et d'une matrice extracellulaire composée majoritairement de collagène, d'élastine et d'une substance, dite substance fondamentale. Ces composants sont synthétisés par les fibroblastes. On y trouve aussi des leucocytes, des mastocytes ou encore des macrophages tissulaires. Enfin, le derme est traversé par des vaisseaux sanguins et des fibres nerveuses.
La cohésion entre l'épiderme et le derme est assurée par la jonction dermo-épidermique.
L'équilibre de la barrière cutanée et des muqueuses est dépendant de mécanismes biologiques complexes faisant intervenir de nombreux facteurs de croissance, hormones et enzymes. Une altération de la barrière cutanée peut notamment se traduire par un trouble de l'hydratation.
La β-endorphine est un membre du groupe des « peptides dérivés de la proopiomélanocortine » (POMC) qui comprend entre autres l'adrenocorticotropic hormone (ACTH), l'α-melanocyte-stimulating hormone ( α MSH) et la β-lipotropic hormone (β-LPH). La β-endorphine est un peptide de 31 acides aminés correspondant à la séquence d'acides aminés 61-91 de la β-LPH, elle même issue du clivage de la POMC.

La POMC et les peptides dérivés de la POMC sont formés principalement au niveau central dans l'hypophyse et l'hypothalamus. La β-endorphine est sécrétée plus précisément par les cellules mélanotrophes présentes dans le lobe antérieur de la glande pituitaire. Ces peptides sont des éléments de l'axe hypothalamo hypophyso adrénalien. Après passage dans la circulation sanguine, ils sont capables d'exercer des effets sur des tissus cibles périphériques très variés. La β-endorphine exerce ses effets biologiques après fixation à des récepteurs de type opiacés. Il existe quatre types de récepteurs opiacés, dénommés mu, delta et kappa et lambda. La β-endorphine présente une affinité forte pour les récepteurs mu et delta. Le tissu cutané humain exprime de façon constitutive un récepteur β-endorphine actif (Bigliardi and coll, 1998). La β-endorphine est en effet un agoniste spécifique de récepteur opiacés de type µ dans la peau humaine. Ces récepteurs sont exprimés dans toutes les couches de l'épiderme avec un marquage plus prononcé au niveau des couches basales. Ils ont été identifiés aussi au niveau des conduits des glandes sudorales, des follicules pileux (Weinstein DD et coll, J. Invest. Dermatol, 2002, 709-710) et des mélanocytes (Kauser S and coll, J. Invest. Dermatol, 2003, 120, 1073-1080).

Outre leur origine centrale, les peptides dérivés de la POMC sont produits par différents tissus périphériques (testicules, ovaires, foie, reins, poumons, tractus gastro-intestinal, cellules du système immunitaire...). Au niveau cutané, il a été montré que certains types cellulaires tels que mélanocytes, kératinocytes, cellules endothéliales, cellules de Langherans et les fibroblastes dermiques sont capables de synthétiser l'ACTH et l'α-MSH. Des stimulis (promoteurs tumoraux, interleukine 1, rayonnement ultra-violet) peuvent d'ailleurs augmenter cette sécrétion dans certaines conditions expérimentales. Il est décrit dans la littérature que différents types cellulaires présents au niveau cutané et en particulier les kératinocytes humains qui constituent l'un des composants essentiels de l'épiderme, sont capables de synthétiser et sécréter la béta-endorphine. Selon ces auteurs, la β-endorphine produite localement serait capable d'exercer des effets biologiques locaux ou généraux.

La demande FR 2 811 228 décrit l'utilisation d'oligosaccharides pour stimuler la production de bêta-endorphines dans la peau; l'objectif des auteurs est de générer une activité relaxante, apaisante, voire même analgésique par la libération de ce peptide au niveau cutané.

Le rôle spécifique de la β-endorphine au niveau cutané et plus particulièrement au niveau épidermique reste peu défini. Une étude récente montre son effet potentiel dans la mélanogénèse par action directe au niveau du mélanocyte. Au niveau du kératinocyte épidermique, Nissen JB et al (Exp. Dermatol. 1997, 6, 222-229) indiquent que la β-endorphine n'a pas d'effet sur la prolifération et la différenciation épidermique.
Dans le présent essai, les inventeurs ont mis en évidence de manière totalement inattendue que la β-endorphine exerce des effets favorisant la différenciation kératinocytaire et la cornéification des kératinocytes, ce qui représente une activité nouvellement décrite. Celle-ci est favorable pour améliorer l'état d'hydratation de la peau et lutter contre l'altération de la barrière cutanée survenant à la suite de stress externes (effets des polluants atmosphériques, des rayonnements ultra-violets...) ou de stress internes dits stress psychologiques. Pour mettre en évidence ces effets sur le kératinocyte humain en culture, les auteurs ont utilisé ont un modèle d'épiderme reconstruit constitué de kératinocytes en multi-couche en état de prolifération, différenciation ou cornéification. Il est bien connu que ce modèle in vitro est adapté pour évaluer les effets d'un actif sur la prolifération et la différenciation kératinocytaire.
L'activité de la β-endorphine dans ce modèle se traduit par l'activation simultanée de trois facteurs essentiels de la différenciation épidermique terminale et de la cornéification des kératinocytes. Ces trois facteurs sont la calmodulin-like skin protein (CLSP), la loricrine et la transglutaminase de type I (TGk). La CLSP est une calciprotéine spécifique de la peau (Mehul B et al, JID, 2000, 275, 17, 12841-7).
Dans la peau humaine normale, la CLSP est exprimée exclusivement dans le stratum granulosum et au niveau des couches inférieures du stratum cornéum. Elle est reconnue aujourd'hui comme un facteur important de la différenciation kératinocytaire. La loricrine est une protéine basique précurseur de l'enveloppe cornée. Elle participe à la formation de structures cornéifiées en s'associant par des ponts disulfures et surtout par des liaisons L glutamine lysine. Elle est un composant majeur des L-granules de kératohyaline de la couche granuleuse et elle est exprimée à un stade tardif de la différenciation kératinocytaire. La TGk est une enzyme appartenant à la famille des transpeptidases. C'est une enzyme calcium-dépendante qui catalyse la formation des ponts isopeptidiques ε(γ-glutamyl) lysine entre protéines épidermiques et favorise donc la formation des enveloppes cornées. La β-endorphine présente donc la propriété d'activer 3 facteurs essentiels et complémentaires de la différenciation terminale du kératinocyte et de sa cornéification. Ce profil particulier est traduit ici comme effet β-endorphine-like.
C'est pourquoi la présente invention a pour objet l'utilisation de β-endorphine ou d'un composé mimétique de la β-endorphine ou β-endorphine-like, dans un procédé de traitement cosmétique pour maintenir et/ou renforcer l'épiderme, en particulier pour maintenir l'intégrité et/ou l'épaisseur de différentes couches de l'épiderme notamment du stratum corneum, et donc la fonction barrière de l'épiderme.
Compte-tenu des éléments pré-cités, il apparaît que les meilleurs moyens pour reproduire les effets de la β-endorphine au niveau épidermique est d'utiliser des actifs désignés dans la présente demande par "β-endorphine like", c'est à dire induisant au niveau cutané, et plus particulièrement dans les kératinocytes humains, les effets de la β-endorphine. On peut soit utiliser la β-endorphine et/ou ses fragments, soit utiliser des actifs mimant les effets de la β-endorphine sur la différenciation du kératinocyte humain.
Par actifs ( ou agents) mimant les effets de la β-endorphine ou agents mimétiques de la β-endorphine au sens de la présente invention, on entend en particulier des substances capables d'induire la production par des kératinocytes d'au moins deux des trois facteurs suivant: la calmodulin-like skin protein (CLSP), la loricrine et la transglutaminase de type I (TGK), de préférence de ces 3 facteurs. Avantageusement, les actifs mimant les effets de la β-endorphine utiles selon l'invention seront déterminés sur un modèle de kératinocytes en culture multi-couche. Les actifs mimétiques de la β-endorphine adaptés à la mise en oeuvre de l'invention augmentent la production de CLSP, de loricrine et/ou de TGK par les kératinocytes en culture de préférence d'au moins 50% par rapport à des kératinocytes témoin cultivés sans le produit mimant la β-endorphine. Les fragments de β-endorphine utiles pour la mise en oeuvre de l'invention seront de préférence des actifs mimant la β-endorphine tels que définis dans ce qui précède.

Les fragments actifs de β-endorphine peuvent être par exemple des séquences peptidiques correspondants à la partie N ou C terminal de béta-endorphine, éventuellement protégés sur la fonction acide ou amine par un groupement approprié. Avantageusement, on utilise des fragments de longueur inférieure ou égale à 10 acides aminés et comportant de préférence au moins 2 acides aminés, notamment d'environ 2 à 8 acides aminés, par exemple de 6 à 8 acides aminés. Les fonctions acides terminales de ces fragments pourront être protégées par des réactions connues de l'homme du métier, notamment par estérification et/ou par formation d'un groupement amide; de même, l'extrémité amino-terminale du fragment de βendorphine pourra être protégé par acylation, notamment par formation d'un groupement acétate. Les fragments obtenus par délétion et/ou substitution d'un ou plusieurs acides aminés des séquences N ou C terminales de β-endorphine tels que définis dans ce qui précède pourront également être utiles dans le cadre de l'invention. Ces fragments peuvent être salifiés par des acides minéraux ou organiques adaptés. On peut citer de manière non limitative les sels tels que les chlorure, sulfate, nitrate, borate, carbonate, gluconate, oxalate, acétate, citrate ou oléate.

Des exemples de tels fragments sont ainsi :

H-Lys-Asn-Ala-His-Lys-Lys-Gly-Gln-OH
Ac-Lys-Asn-Ala-His-Lys-Lys-Gly-Gln-OEt
Ac-Lys-Asn-Ala-His-Lys-Lys-Gly-Gln-NH2
H-Ala-His-Lys-Lys-Gly-Gln-OH
Ac-Ala-His-Lys-Lys-Gly-Gln-OEt
Ac-Ala-His-Lys-Lys-Gly-Gln-OH
H-Lys-Gly-Gln-OH
Ac-Lys-Gly-Gln-OEt
Ac-Lys-Gly-Gln-NH2

H-Tyr-Gly-Gly-Phe-Met-Thr-Ser-Glu-OH
Ac-Tyr-Gly-Gly-Phe-Met-Thr-Ser-Glu-OEt
Ac-Tyr-Gly-Gly-Phe-Met-Thr-Ser-Glu-NH2
H-Tyr-Gly-Gly-Phe-Met-Thr-OH
Ac-Tyr-Gly-Gly-Phe-Met-Thr-OEt
Ac-Tyr-Gly-Gly-Phe-Met-Thr-NH2
H-Tyr-Gly-Gly-Phe-Met-OH
Ac-Tyr-Gly-Gly-Phe-Met-NH2
Ac-Tyr-Gly-Gly-Phe-Met-OEt
H-Tyr-Gly-Gly-OH
Ac-Tyr-Gly-Gly-OEt
AcTyr-Gly-Gly-NH2

De manière inattendue, les inventeurs ont mis en évidence qu'un extrait de fève de cacao riche en polyphénols et bases xanthiques, en particulier en théobromine, a un profil d'activité tout point comparable à celui de la β-endorphine .

C'est pourquoi l'un des objets de la présente invention est l'utilisation de β-endorphine, de l'un de ses fragments et/ou d'un agent capable de mimer l'activité de la β-endorphine sur les kératinocytes de la peau pour favoriser l'hydratation de la peau.

Selon un autre aspect; l'invention concerne l'utilisation de β-endorphine, de l'un de ses fragments ou d'un agent capable de mimer l'activité de la β-endorphine sur les kératinocytes de la peau pour améliorer la fonction barrière de la peau.
Selon encore un autre aspect, l'invention concerne l'utilisation de β-endorphine, de l'un de ses fragments et/ou d'un agent capable de mimer l'activité de la β-endorphine sur les kératinocytes de la peau, pour renforcer la résistance de la peau aux agressions extérieures, en particulier à la pollution et aux agents polluants (tels que les particules, l'ozone, les oxydes d'azote, les oxydes de soufre et/ou les métaux lourds comme notamment le cobalt, le cadmium ou le mercure) ou aux radicaux libres, aux variations brusques de température ou d'humidité relative de l'atmosphère, au vent, à l'air conditionné, aux contraintes mécaniques, notamment à l'irritation provoquée par certains tissus rugueux ou des soins d'hygiène trop agressifs; cette utilisation peut également viser à renforcer la résistance de la peau aux stress internes, notamment aux modifications induites par des stress psychologiques ou la fatigue qui ont un retentissement sur l'état de surface de la peau.

La β-endorphine et/ou les agents β-endorphine like selon l'invention seront ainsi particulièrement utiles pour stimuler l'hydratation naturelle et l'éclat de la peau, qui parait plus lumineuse et plus douce, et pour améliorer et/ou diminuer le micro-relief de la peau.

Les agents β-endorphine like utiles selon l'invention pourront être obtenus par synthèse chimique ou par extraction à partir de sources naturelles, notamment à partir de végétaux, par des méthodes connues de l'homme du métier et notamment par des solvants aqueux, alcooliques, et/ou hydro-alcooliques. Des extraits adaptés sont notamment des extraits contenant des bases xanthiques et des polyphénols, en particulier des flavonoïdes et des anthocyanes.
Avantageusement, l'agent β-endorphine like selon l'invention est un extrait d'un plante de la famille des Sterculiacées, en particulier de Theobroma cacao. On peut citer plus particulièrement des extraits obtenus à partir de coques de fèves de cacao, par exemple par extraction hydro-glycolique, tel celui commercialisé par la société Solabia sous la dénomination Caobromine®. De tels extraits sont titrés en bases xanthiques, ils contiennent au moins 1% et notamment au moins 1,5% de caféine, et au moins 0,01%, notamment au moins 0,05% de théobromine. Ils contiennent également des flavonoïdes et du magnésium, ce dernier élément à raison d'environ 49-50ppm
De tels extraits sont connus en tant qu'agents amincissants.

L'invention a donc également pour objet un procédé de traitement cosmétique pour maintenir ou favoriser l'hydratation de la peau et/ou des muqueuses, renforcer la fonction barrière de la peau et/ou des muqueuses, et favoriser la résistance de la peau et/ou des muqueuses aux agressions externes liées à l'environnement ou aux modifications provoquées par des stress intrinsèques, comprenant l'application d'une quantité efficace de β-endorphine, de l'un de ses fragments et/ou d'un agent capable de mimer l'activité de la β-endorphine sur les kératinocytes de la peau. L'agent sera appliqué sur la partie de la peau à traiter, en particulier sur le visage, le cou ou les mains, de façon quotidienne ou pluriquotidienne; l'application sera renouvelée tous les jours pendant une période variable selon les effets souhaités, généralement de 3 à 6 semaines, mais pourra être prolongée ou poursuivie en continu.
L'invention a également pour objet l'utilisation cosmétique, dans une composition contenant un milieu physiologiquement acceptable, d'au moins un composé choisi parmi la β-endorphine et les agents mimétiques de la β-endorphine, comme agent pour favoriser la différenciation et/ou la cornéification des kératinocytes; en particulier, elle vise l'utilisation comme agent pour maintenir et/ou renforcer l'épiderme, pour maintenir l'intégrité et/ou l'épaisseur des différentes couches de l'épiderme en particulier du stratum corneum, pour maintenir et/ou améliorer la fonction barrière de la peau, et/ ou pour favoriser l'hydratation naturelle de la peau.
Les agents mimétiques de la β-endorphine peuvent notamment être des fragments de cette molécule, tels que définis précédemment. La β-endorphine et/ou les agents mimétiques de la β-endorphine peuvent être utilisés seuls ou en mélange en toutes proportions.

Un autre objet de l'invention est l'utilisation d'au moins un composé choisi parmi la β-endorphine et les agents mimétiques de la β-endorphine sur les kératinocytes de la peau pour la préparation d'une composition cosmétique et/ou dermatologique destinée à lutter contre les altérations de la fonction barrière et/ou les modifications de l'intégrité du stratum corneum.
De préférence, la β-endorphine ou les agents mimétiques de la β-endorphine ne sont pas destinés à lutter contre les phénomènes d'hypersensibilisation liés à la présence de nickel.

La β-endorphine et/ou les agents β-endorphine like selon l'invention, seront notamment formulés dans des compositions adaptées à une application topique sur la peau, c'est à dire dans des compositions à usage cosmétique ou dermatologique. La quantité sera adaptée par l'homme du métier selon l'actif utilisé, mais sera généralement d'au moins 0,0001% et pourra par exemple varier de 0,001% à 10%, notamment de 0,01 à 5% en poids par rapport au poids total de la composition. Par exemple, dans le cas d'un extrait de coque de fève de cacao, on utilisera de 0,1 à 5% en poids d'extrait dilué à 3% dans le solvant d'extraction; avantageusement, les quantités de cette dilution dans la composition seront d'environ 0,5 à 2%, plus particulièrement de 1 à 1,5% en poids.

Cette composition peut être plus ou moins fluide et avoir l'aspect d'une crème blanche ou colorée, d'une pommade, d'un lait, d'une lotion, d'un sérum, d'une pâte, d'une mousse. Elle peut également se présenter sous forme solide, en particulier sous forme de stick. Elle peut être utilisée comme produit de soin et/ou comme produit de maquillage pour la peau.

La composition selon l'invention peut se présenter sous toutes les formes galéniques normalement utilisées dans le domaine cosmétique, et elle peut être notamment sous forme d'une solution huileuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles. On préfère utiliser selon cette invention une composition sous la forme d'une émulsion huile-dans-eau.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les hydrocarbures d'origine minérale (huile minérale) ou synthétique (huile de vaseline, isohexadécane), les huiles d'origine végétale (huile d'amande d'abricot, fraction liquide de beurre de karité, huile d'avocat, huile de soja), les huiles d'origine animale (lanoline), les huiles de synthèse (perhydrosqualène, tétraoctanoate de pentaérythrityle), les huiles siliconées (cyclopentasiloxane et cyclohexasiloxane) et les huiles fluorées (perfluoropolyéthers). On peut aussi utiliser comme matières grasses des alcools gras (alcool cétylique ou stéarylique), des acides gras (acide stéarique), des cires (cire de carnauba, ozokérite, cire d'abeille).

Comme émulsionnants et coémulsionnants utilisables dans l'invention, on peut citer par exemple les esters d'acide gras et de polyéthylène glycol tels que le stéarate de PEG-100 et le stéarate de PEG-20 et les esters d'acide gras et de glycérine tels que le stéarate de glycéryle.

De façon connue, la composition utilisée selon l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées de la β-endorphine ou des agents mimant l'activité de la β-endorphine sur les kératinocytes.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme charges, on peut citer par exemple, les particules de polyamide (Nylon) sous forme sphérique ou sous forme de microfibres ; les microsphères de polyméthacrylate de méthyle ; les poudres de copolymère éthylène-acrylate ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate ; les microbilles de résine de silicone telles que celles commercialisées sous la dénomination TOSPEARL par la société Toshiba Silicone ; la silice ; les oxydes métalliques tels que le dioxyde de titane ou l'oxyde de zinc ; le mica ; et leurs mélanges.

La composition utilisée selon l'invention peut en outre contenir d'autres actifs, et notamment au moins un composé choisi parmi : les agents hydratants ; les agents dépigmentants ; les agents anti-glycation ; les inhibiteurs de NO-synthase ; les agents stimulant la synthèse de macromolécules dermiques ou épidermiques et/ou empêchant leur dégradation ; les agents stimulant la prolifération des fibroblastes et/ou des kératinocytes ou stimulant la différenciation des kératinocytes ; les agents myorelaxants ; les agents tenseurs ; les agents anti-pollution et/ou anti-radicalaire, les filtres solaires, et leurs mélanges.

Par "agent hydratant", on entend :
- soit un composé agissant sur la fonction barrière, en vue de maintenir l'hydratation du stratum corneum, ou un composé occlusif. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ;
- soit un composé augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine ;
- soit un composé activant les glandes sébacées tel que la DHEA, ses dérivés 7-oxydés et/ou 17-alkylés, les sapogénines et la vitamine D et ses dérivés.

Les agents dépigmentants susceptibles d'être incorporés dans la composition selon la présente invention comprennent par exemple les composés suivants : l'acide kojique ; l'acide ellagique ; l'arbutine et ses dérivés tels que ceux décrits dans les demandes EP-895 779 et EP-524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique ou procystéine, ainsi que ses sels et esters ; l'acide ascorbique et ses dérivés, notamment le glucoside d'ascorbyle ; et les extraits de plantes, en particulier de réglisse, de mûrier et de scutellaire, sans que cette liste soit limitative.

Par "agent anti-glycation", on entend un composé prévenant et/ou diminuant la glycation des protéines de la peau, en particulier des protéines du derme telles que le collagène.

Des exemples d'agents anti-glycation sont les extraits végétaux de la famille des Ericaceae, tels qu'un extrait de myrtille *(Vaccinium angustifolium)* ; l'ergothionéine et ses dérivés ; et les hydroxystilbènes et leurs dérivés, tels que le resvératrol et le 3,3', 5,5'-tétrahydroxystilbène.

Des exemples d'inhibiteurs de NO-synthase convenant à une utilisation dans la présente invention comprennent notamment un extrait de végétal de l'espèce *Vitis vinifera* qui est notamment commercialisé par la société Euromed sous la dénomination Leucocyanidines de raisins extra, ou encore par la société Indena sous la dénomination Leucoselect®, ou enfin par la société Hansen sous la dénomination Extrait de marc de raisin ; un extrait de végétal de l'espèce *Olea europaea* qui est de préférence obtenu à partir de feuilles d'olivier et est notamment commercialisé par la société VINYALS sous forme d'extrait sec, ou par la société Biologia & Technologia sous la dénomination commerciale Eurol BT ; et un extrait d'un végétal de l'espèce *Gingko biloba* qui est de préférence un extrait aqueux sec de ce végétal vendu par la société Beaufour sous le nom commercial Ginkgo biloba extrait standard.

Parmi les actifs stimulant les macromolécules du derme ou empêchant leur dégradation, on peut citer ceux qui agissent :
- soit sur la synthèse du collagène, tels que les extraits de Centella asiatica ; les asiaticosides et dérivés ; l'acide ascorbique ou vitamine C et ses dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; et les hormones végétales telles que les auxines et les lignanes.
- soit sur la synthèse d'élastine, tels que l'extrait de Saccharomyces Cerivisiae commercialisé par la société LSN sous la dénomination commerciale Cytovitin® ; et l'extrait d'algue Macrocystis pyrifera commercialisé par la société SECMA sous la dénomination commerciale Kelpadelie® ;
- soit sur la synthèse des glycosaminoglycanes, tels que le produit de fermentation du lait par lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3® ; et l'extrait de Saccharomyces cerevisiae disponible notamment auprès de la société SILAB sous la dénomination commerciale Firmalift® ou auprès de la société LSN sous la dénomination commerciale Cytovitin® ;
- soit sur la synthèse de la fibronectine, tels que l'extrait de zooplancton Salina commercialisé par la société SEPORGA sous la dénomination commerciale GP4G® ; l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil® ;
- soit sur l'inhibition des métalloprotéinases (MMP) telles que plus particulièrement les MMP 1, 2, 3, 9. On peut citer : les rétinoïdes et dérivés, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon ou de sauge ;
- soit sur l'inhibition des sérine protéases telles que l'élastase leucocytaire ou la cathepsine G. On peut citer : l'extrait peptidique de graines de légumineuse (Pisum sativum) commercialisé par la société LSN sous la dénomination commerciale Parelastyl® ; les héparinoïdes ; et les pseudodipeptides tels que l'acide {2-[acétyl-(3-trifluorométhyl-phényl)-amino]-3-méthyl-butyrylamino} acétique.

Parmi les actifs stimulant les macromolécules épidermiques, telles que la fillagrine et les kératines, on peut citer notamment l'extrait de lupin commercialisé par la société SILAB sous la dénomination commerciale Structurine® ; l'extrait de bourgeons de hêtre Fagus sylvatica commercialisé par la société GATTEFOSSE sous la dénomination commerciale Gatuline® ; et l'extrait de zooplancton Salina commercialisé par la société SEPORGA sous la dénomination commerciale GP4G®.

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; et les hormones végétales telles que les giberrellines et les cytokinines.

Les agents stimulant la prolifération des kératinocytes, utilisables dans la composition selon l'invention, comprennent notamment les rétinoïdes tels que le rétinol et ses esters, dont le palmitate de rétinyle ; le phloroglucinol ; les extraits de tourteaux de noix commercialisés par la société GATTEFOSSE ; et les extraits de Solanum tuberosum commercialisés par la société SEDERMA.

Les agents stimulant la différenciation des kératinocytes comprennent par exemple les minéraux tels que le calcium ; l'extrait de lupin commercialisé par la société SILAB sous la dénomination commerciale Photopréventine®; le beta-sitosteryl sulfate de sodium commercialisé par la société SEPORGA sous la dénomination commerciale Phytocohésine®; et l'extrait de maïs commercialisé par la société SOLABIA sous la dénomination commerciale Phytovityl® ; et les lignanes tels que le sécoisolaricirésinol.

La composition selon l'invention peut comprendre des agents dermo-décontractants, parmi lesquels on peut citer en particulier l'alvérine et ses sels, notamment le citrate d'alvérine, les sapogénines telles que la diosgénine et les extraits naturels en contenant (tels que les extraits de Wild Yam), certaines amines secondaires et tertiaires carbonylées, des sels organiques ou inorganiques de métaux, en particulier le gluconate de manganèse, l'adénosine, ainsi que l'hexapeptide argireline R commercialisé par la société LIPOTEC. On peut également citer certaines compositions parfumantes à effet dermo-décontractant.

Par "agent tenseur", on entend un composé capable d'exercer une traction sur la peau, qui a pour effet d'estomper temporairement les irrégularités de la surface de la peau, telles que les rides et ridules.

Par l'expression "agent anti-pollution", on entend tout composé capable de piéger l'ozone, les composés aromatiques mono- ou polycycliques tels que le benzopyrène et/ou les métaux lourds tels que le cobalt, le mercure, le cadmium et/ou le nickel. Par "agent anti-radicalaire", on entend tout composé capable de piéger les radicaux libres.

Comme agents piégeurs d'ozone utilisables dans la composition selon l'invention, on peut citer en particulier la vitamine C et ses dérivés dont le glucoside d'ascorbyle ; les phénols et polyphénols, en particulier les tannins, l'acide ellagique et l'acide tannique ; l'épigallocatéchine et les extraits naturels en contenant ; les extraits de feuille d'olivier ; les extraits de thé, en particulier de thé vert ; les anthocyanes ; les extraits de romarin ; les acides phénols, en particulier l'acide chorogénique ; les stilbènes, en particulier le resvératrol ; les dérivés d'acides aminés soufrés, en particulier la S-carboxyméthylcystéine ; l'ergothionéine ; la N-acétylcystéine ; des chélatants comme la N,N'-bis-(3,4,5-triméthoxybenzyl)éthylènediamine ou l'un de ses sels, complexes métalliques ou esters ; des caroténoïdes tels que la crocétine ; et des matières premières diverses comme le mélange d'arginine, ribonucléate d'histidine, mannitol, adénosinetriphosphate, pyridoxine, phénylalanine, tyrosine et ARN hydrolysé commercialisé par les Laboratoires Sérobiologiques sous la dénomination commerciale CPP LS 2633-12F®, la fraction hydrosoluble de maïs commercialisée par la société SOLABIA sous la dénomination commerciale Phytovityl®, le mélange d'extrait de fumeterre et d'extrait de citron commercialisé sous la dénomination Unicotrozon C-49® par la société Induchem, et le mélange d'extraits de ginseng, de pomme, de pêche, de blé et d'orge vendu par la société PROVITAL sous la dénomination commerciale Pronalen Bioprotect®.

Comme agents piégeurs de composés aromatiques mono- ou polycycliques utilisables dans la composition selon l'invention, on peut citer en particulier les tannins tels que l'acide ellagique ; les dérivés indoles, en particulier l'indol-3-carbinol ; les extraits de thé en particulier de thé vert, les extraits de Jacinthe d'eau ou eichornia crassipes ; et la fraction hydrosoluble de maïs commercialisée par la société SOLABIA sous la dénomination commerciale Phytovityl®.

Enfin, comme agents piégeurs de métaux lourds utilisables dans la composition selon l'invention, on peut citer en particulier les agents chélatants tels que l'EDTA, le sel pentasodique d'éthylènediamine tétraméthylène phosphonique, et la N,N'-bis-(3,4,5-triméthoxybenzyl)éthylènediamine ou l'un de ses sels, complexes métalliques ou esters ; l'acide phytique ; les dérivés de chitosan ; les extraits de thé, en particulier de thé vert ; les tannins tels que l'acide ellagique ; les acides aminés soufrés tels que la cystéine ; les extraits de Jacinthe d'eau (Eichornia crassipes) ; et la fraction hydrosoluble de maïs commercialisée par la société SOLABIA sous la dénomination commerciale Phytovityl®.

Les agents anti-radicalaires utilisables dans la composition selon l'invention comprennent, outre certains agents anti-pollution mentionnés précédemment, la vitamine E et ses dérivés tels que l'acétate de tocophéryle ; les bioflavonoïdes ; le co-enzyme Q10 ou ubiquinone ; certaines enzymes comme la catalase, le superoxyde dismutase, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; les lignanes ; et la mélatonine.

La composition selon l'invention peut également renfermer des filtres ou agents photoprotecteurs UVA et/ou UVB, sous forme de composés organiques et/ou inorganiques, hydrosolubles ou liposolubles ou bien insolubles dans les solvants cosmétiques couramment utilisés.

Les agents photoprotecteurs organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US5,237,071, US5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples d'agents photoprotecteurs actifs dans l'UV-A et/ou l'UV-B, on peut citer désignés ci-dessus sous leur nom INCl:
- les dérivés de l'acide para-aminobenzoïique, dont les suivants : PABA, Ethyl PABA, Ethyl Dihydroxypropyl PABA, Ethylhexyl Diméthyl PABA vendu notamment sous le nom « ESCALOL 507 » par ISP, Glyceryl PABA, PEG-25 PABA vendu sous le nom « UVINUL P25 » par BASF,
- les dérivés salicyliques, dont les suivants : Homosalate vendu notamment sous le notamment sous le nom « NEO HELIOPAN OS » par Haarmann et REIMER, Dipropyleneglycol Salicylate vendu notamment sous le nom « DIPSAL » par SCHER, TEA Salicylate vendu notamment sous le nom « NEO HELIOPAN TS » par Haarmann et REIMER,
- les dérivés du dibenzoylméthane, dont les suivants : Butyl Methoxydibenzoylmethane vendu notamment sous le nom commercial « PARSOL 1789 » par HOFFMANN LAROCHE, Isopropyl Dibenzoylmethane,
- les dérivés cinnamiques, dont les suivants :Ethylhexyl Methoxycinnamate vendu notamment sous le nom commercial « PARSOL MCX » par HOFFMANN LAROCHE, Isopropyl Methoxy cinnamate, Isoamyl Methoxy cinnamate vendu notamment sous le nom commercial « NEO HELIOPAN E 1000 » par HAARMANN et REIMER, Cinoxate, DEA Methoxycinnamate, Diisopropyl Methylcinnamate, Glyceryl Ethylhexanoate Dimethoxycinnamate,
- les dérivés de β,β'-diphénylacrylate, dont les suivants : Octocrylene vendu notamment sous le nom commercial « UVINUL N539 » par BASF, Etocrylene vendu notamment sous le nom commercial « UVINUL N35 » par BASF,
- les dérivés de la benzophénone, dont les suivants : Benzophenone-1 vendue notamment sous le nom commercial « UVINUL 400 » par BASF, Benzophenone-2 vendue notamment sous le nom commercial « UVINUL D50 » par BASF, Benzophenone-3 ou Oxybenzone vendue notamment sous le nom commercial « UVINUL M40 » par BASF, Benzophenone-4 vendue notamment sous le nom commercial « UVINUL MS40 » par BASF, Benzophenone-5, Benzophenone-6 vendue notamment sous le nom commercial « Helisorb 11 » par Norquay, Benzophenone-8 vendue notamment sous le nom commercial « Spectra-Sorb UV-24 » par American Cyanamid, Benzophenone-9 vendue notamment sous le nom commercial« UVINUL DS-49» par BASF, Benzophenone-12, et le 2-(4-diéthylamino-2-hydroxybenzoyl)-benzoate de n-hexyle
- les dérivés du benzylidène camphre, dont les suivants : 3-Benzylidene camphor, 4-Methylbehzylidene camphor vendu notamment sous le nom « EUSOLEX 6300 » par MERCK , Benzylidene Camphor Sulfonic Acid, Camphor Benzalkonium Methosulfate, Terephthalylidene Dicamphor Sulfonic Acid, Polyacrylamidomethyl Benzylidene Camphor,
- les dérivés de benzimidazole, dont les suivants : Phenylbenzimidazole Sulfonic Acid vendu notamment sous le nom commercial « EUSOLEX 232 » par MERCK, Disodium Phenyl Dibenzimidazole Tetra-sulfonate vendu notamment sous le nom commercial commercial « NEO HELIOPAN AP » par Haarmann et REIMER,
- les dérivés de triazine, dont les suivants : Anisotriazine vendu notamment sous le nom commercial «TINOSORB S » par CIBA SPECIALTY CHEMICALS, Ethylhexyl triazone vendu notamment sous le nom commercial «UVINUL T150 » par BASF, Diethylhexyl Butamido Triazone vendu notamment sous le nom commercial « UVASORB HEB » par SIGMA 3V, et la 2,4,6- tris-(4'amino-benzalmalonate de diisobutyle)-s-triazine,
- les dérivés de benzotriazole, dont les suivants : Drometrizole Trisiloxane vendu sous le nom « Silatrizole » par RHODIA CHIMIE, Methylène bis-Benzotriazolyl Tetramethylbutylphénol, vendu notamment sous forme solide sous le nom commercial « MIXXIM BB/100 » par FAIRMOUNT CHEMICAL ou sous forme micronisée en dispersion aqueuse sous le nom commercial « TINOSORB M » par CIBA SPECIALTY CHEMICALS,
- les dérivés anthranilique, dont le Menthyl anthranilate vendu sous le nom commercial commercial « NEO HELIOPAN MA » par Haarmann et REIMER,
- les dérivés d'imidazolines, dont l'Ethylhexyl Dimethoxybenzylidene Dioxoimidazoline Propionate,
- les dérivés de benzalmalonate, dont le polyorganosiloxane à fonctions benzalmalonate vendu sous la dénomination commerciale « PARSOL SLX » par HOFFMANN LAROCHE,
- les dérivés de 4,4-diarylbutadiène, dont le 1,1-dicarboxy (2,2'-diméthyl-propyl)-4,4-diphénylbutadiène,
- et leurs mélanges.

Les agents photoprotecteurs organiques plus particulièrement préférés sont choisis parmi les composés suivants : Ethylhexyl Salicylate, Ethylhexyl Methoxycinnamate, Octocrylene, Phenylbenzimidazole Sulfonic Acid, Benzophenone-3, Benzophenone-4, Benzophenone-5, 4-Methylbenzylidene camphor, Terephthalylidene Dicamphor Sulfonic Acid, Disodium Phenyl Dibenzimidazole Tetra-sulfonate, la 2,4,6-tris-(4'-amino benzalmalonate de diisobutyle)-s-triazine, Anisotriazine, Ethylhexyl triazone, Diethylhexyl Butamido Triazone, Méthylène bis-Benzotriazolyl Tetramethylbutylphénol, Drometrizole Trisiloxane, 1,1-dicarboxy (2,2'-diméthyl-propyl)-4,4-diphénylbutadiène, et leurs mélanges.

Les agents photoprotecteurs inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les agents photoprotecteurs sont généralement présents dans les compositions selon l'invention dans des proportions allant de 0,1 à 20% en poids par rapport au poids total de la composition, et de préférence allant de 0,2 à 15% en poids par rapport au poids total de la composition.

Les exemples qui suivent sont destinés à illustrer l'invention.

### Exemple 1: Mise en évidence de l'activité sur les kératinocytes

### Etudes expérimentales

L'étude a été réalisée sur modèle d'épiderme reconstruit Skinethic. Dès réception, les épidermes ont été pré-cultivés pendant 24 heures en milieu SkinEthic. Le milieu de culture a ensuite été remplacé par du milieu contenant le produit à l'essai (deux épidermes par concentration testée), puis les épidermes ont été cultivés pendant 24 heurs à 37°C et 5% de CO₂. Un lot de 2 épidermes n'a pas été traité. Les surnageants de culture ont ensuite été éliminés et les épidermes ont été rincés avec une solution de PBS, découpés et déposés dans des tubes stériles RNA-free en présence de Tri-Reagent (Sigma T9424) puis immédiatement congelés à -80°C. Les marqueurs dont l'expression a été recherchée sont reportés dans le tableau ci-dessous.
Les couples de primers qui ont été utilisés dans cette étude permettent l'amplification des fragments spécifiques suivants :

| Marqueurs sélectionnés | abréviation | Genbank access |
|---|---|---|
| Transglutaminase 1 | TGK | X57974 |
| Loricrine | LORI | M61120 |
| Calmodulin-like skin protein | CLSP | AF172852 |

La Réverse Transcription a été réalisée de la façon suivante :
- extraction des ARN totaux de chaque épiderme à l'aide de Tri-reagent selon le protocole préconisé par le fournisseur, puis nouvelle extraction par du chloroforme et précipitation à l'isopropanol.
- élimination des traces d'ADN potentiellement contaminant par traitement avec le système DNA-free (Ambion).
- réalisation de la réaction de Réverse-Transcription de l'ARNm en présence de l'amorce oligo(dT) et de l'enzyme Superscript II (Gibco)
- quantification par fluorescence d l'ADNc synthétisé et ajustement des concentrations de 50 ng/ml.

Les réactions de PCR (polymerase chain reactions) ont été réalisées par PCR quantitative avec le système « Light cycler » (Roche Molecular Systems Inc.) selon les procédures recommandées par le fournisseur.

Les conditions de PCR ont été les suivantes : activation de 10 min à 95 °C, réactions de PCR (10 sec à 95 °C, 5 sec à 64 °C et 35 sec à 72 °C) 40 cycles, fusion de 5 sec à 95 °C puis 5 sec à 60 °C.

L'incorporation de fluorescence dans l'ADN amplifié a été mesurée en continu au cours des cycles de PCR. Ce système permet d'obtenir des courbes de mesure de la fluorescence en fonction des cycles de PCR et d'évaluer ainsi une valeur d'expression relative pour chaque marqueur. La valeur de RE (relative expression) est exprimée en unités arbitraires selon la formule suivante : (1/2 ^{nombre de cycles}) × 10⁶.

### Résultats

Les résultats ont été rapportés à la quantité de messager actine (gène de référence) et sont représentés, par marqueur dans les tableaux suivants.

| Marqueur Transglutaminase I (TGK) | | | | | |
|---|---|---|---|---|---|
| Traitement | Concentration | RE*Actine (UA) | RE*TGK (UA) | TGK/Actine | % témoin |
| Témoin | - | 1.11 | 0.094 | 0.085 | 100 |
| Beta-endorphine | 0.01 µM | 1.11 | 0.165 | 0.149 | 176 |
| Extrait de fève de cacao | 0.1% | 1.10 | 0.22 | 0.201 | 238 |

La Caobromine à la concentration la plus élevée entraîne une augmentation nette de l'expression du messager de la TGK. La β endorphine, à concentration physiologique induit une effet de même type bien que légèrement moins prononcé.

| Marqueur Camodulin like skin protein (CLSP) | | | | | |
|---|---|---|---|---|---|
| Traitement | Concentration | RE*Actine (UA) | RE*CLSP (UA) | CLSP/Actine | % témoin |
| Témoin | - | 1.04 | 0.0574 | 0.0552 | 100 |
| Beta-endorphine | 0.01 µM | 1.07 | 0.1032 | 0.0961 | 174 |

| Traitement | Concentration | RE*Actine (UA) | RE*CLSP (UA) | CLSP/Actine | % témoin |
|---|---|---|---|---|---|
| Témoin | - | 0.94 | 0.06 | 0.061 | 100 |
| Extrait de fève de cacao | 0.1 % | 0.94 | 0.17 | 0.178 | 291 |

Les deux produits, quelque soit la concentration utilisée induisent une augmentation de l'expression du messager de la CLSP.

| Marqueur Loricrine | | | | | |
|---|---|---|---|---|---|
| Traitement | Concentration | RE*Actine (UA) | RE*LORI (UA) | LORI/Actine | % témoin |
| Témoin | - | 1.09 | 6.19 | 5.67 | 100 |
| Beta-endorphine | 0.01 µM | 1.11 | 11.24 | 10.2 | 179 |
| Extrait de fève de cacao | 0.1% | 1.06 | 16.13 | 15.19 | 268 |

La β endorphine à concentration physiologique et la Caobromine aux deux concentrations testées stimulent l'expression de ce marqueur.

### Exemple 2 : Préparation d'une émulsion H/E

| **Emulsion HIE** | |
|---|---|
| Phase A (phase huileuse): | |
| Melange d'arachidyl polyglucoside et d'alcools arachidique et | |
| behenique (15/85) (Montanov 202 de la société SEPPIC) | 1.5% |
| Beurre de Karité | 1% |
| Melange de mono-di-stéarate de glycéryle et de stéarate de | |
| potassium (Tegin de la société Goldschmidt) | 0.5% |
| Triglycérides capryliques/ capriques | 4% |
| Cyclopentadimethylsiloxane | 6% |

| Phase B (phase aqueuse) | |
|---|---|
| Glycerine | 5% |
| Copolymère d'acrylamide (Hostacerin AMPS®s de Clariant) | 1 % |
| Conservateur | qs |
| Eau | qs 100% |

| Phase C | |
|---|---|
| Extrait de coque de feves de cacao (Theobroma cacao) | 1 % |
| dans Eau/Butylene Glycol Stab. (soit 3.3% de Matière active) | |

| Phase D | |
|---|---|
| Parfum | qs |

### Mode opératoire:

On prépare les phases les phases A et B à chaud (environ 80°C) et on incorpore la phase A dans la phase B sous agitation. Après refroidissement à 40 °C on ajoute sous faible cisaillement, la phase C et la Phase D

### Exemple 3: composition pour peaux sèches

La composition est préparée selon la formule suivante:
- Filtre UV 0,1000%
- pigments et nacres 0,2000%
- alcool éthylique 3,5000%
- séquestrant 0,1000%
- acétate de tocophéryle 0,1000%
- sesquistéarate de méthyl-glucose oxyéthyléné 2,5000%
- huiles végétales 2,5000%
- hyaluronate de sodium 0,0200%
- glycérine 7,0000%
- conservateurs 0,8000%
- méthyl-4 esculétol monoéthanoate de sodium 0,1000%
- parfum 0,1500%
- cyclohexa diméthylsiloxane 6,0000%
- triglycérides d'acides caprylique-caprique 6,0000%
- charges 2,0000%
- épaississants et gélifiants 7,0000%
- copolymères bloc [polydiméthylsiloxane][éthyl] [polydiméthylsiloxane] 0,5000%
- Extrait de coque de fèves de cacao (Theobroma cacao) dans Eau/Butylène Glycol 1,0000%
- eau q.s.p. 100%

### Exemple 4 : Effet sur l'hydratation

La composition de l'exemple 3 est évaluée selon le protocole suivant:
Nombre de sujets : 25 sujets
Méthode d'évaluation : cornéomètre
Temps de mesures :
   - T0, avant la première application du produit sur l'avant-bras
   - T4semaines, après la dernière application du produit sur l'avant-bras datant de la veille

### Résultats :

| | Témoin | Traité |
|---|---|---|
| T0 | 29.4 +/- 6.0 | 29.4 +/- 6.2 |
| T 4s | 29.2 +/- 5.9 | 40.8 +/- 8.7 |

### Gain d'hydratation :

T4s : **+39.5%** p<0.0001 (S)

### Conclusion :

Dans les conditions de l'étude, et par rapport à une zone témoin, le soin selon l'invention a un effet hydratant significatif après 4 semaines d'utilisation biquotidienne, le gain d'hydratation est de +39.5%.

## Revendications

1. Utilisation d'au moins un composé choisi parmi la β-endorphine, et les agents mimétiques de la β-endorphine sur les kératinocytes de la peau, dans une composition cosmétique pour améliorer la fonction barrière de la peau.

2. Utilisation d'au moins un composé choisi parmi la β-endorphine et les agents mimétiques de la β-endorphine sur les kératinocytes de la peau, dans une composition cosmétique pour améliorer l'hydratation de la peau.

3. Utilisation d'au moins un composé choisi parmi la β-endorphine, et les agents mimétiques de la β-endorphine sur les kératinocytes de la peau dans une composition cosmétique pour renforcer la résistance de la peau aux stress internes et/ou externes.

4. Utilisation d'au moins un composé choisi parmi la β-endorphine, et les agents mimétiques de la β-endorphine sur les kératinocytes de la peau selon la revendication 3, comme agent renforçant la résistance de la peau à la pollution.

5. Utilisation d'au moins un composé choisi parmi la β-endorphine, et les agents mimétiques de la β-endorphine sur les kératinocytes de la peau pour améliorer l'éclat de la peau.

6. Utilisation selon l'une des revendications précédentes, **caractérisé en ce que** les agents mimétiques de la β-endorphine sur les kératinocytes de la peau sont choisis parmi les fragments de la β-endorphine et les extraits végétaux.

7. Utilisation selon l'une des revendications précédentes **caractérisée en ce que** les agents mimétiques de la β-endorphine sur les kératinocytes de la peau activent la production par les kératinocytes d'au moins deux facteurs choisis parmi la calmodulin-like skin protein (CLSP), la loricrine et la transglutaminase de type I (TGK),

8. Utilisation selon l'une des revendications 6 ou 7 **caractérisée en ce que** les fragments de β-endorphine sont choisis parmi :

H-Lys-Asn-Ala-His-Lys-Lys-Gly-Gln-OH
Ac-Lys-Asn-Ala-His-Lys-Lys-Gly-Gln-OEt
Ac-Lys-Asn-Ala-His-Lys-Lys-Gly-Gln-NH2
H-Ala-His-Lys-Lys-Gly-Gln-OH
Ac-Ala-His-Lys-Lys-Gly-Gln-OEt
Ac-Ala-His-Lys-Lys-Gly-Gln-OH
H-Lys-Gly-Gln-OH

Ac-Lys-Gly-Gln-OEt
Ac-Lys-Gly-Gln-NH2

H-Tyr-Gly-Gly-Phe-Met-Thr-Ser-Glu-OH
Ac-Tyr-Gly-Gly-Phe-Met-Thr-Ser-Glu-OEt
Ac-Tyr-Gly-Gly-Phe-Met-Thr-Ser-Glu-NH2
H-Tyr-Gly-Gly-Phe-Met-Thr-OH
Ac-Tyr-Gly-Gly-Phe-Met-Thr-OEt
Ac-Tyr-Gly-Gly-Phe-Met-Thr-NH2
H-Tyr-Gly-Gly-Phe-Met-OH
Ac-Tyr-Gly-Gly-Phe-Met-NH2
Ac-Tyr-Gly-Gly-Phe-Met-OEt
H-Tyr-Gly-Gly-OH
Ac-Tyr-Gly-Gly-OEt
AcTyr-Gly-Gly-NH2

9. Utilisation selon l'une des revendications 6 ou 7 **caractérisée en ce que** les extraits végétaux mimétiques de la β-endorphine sont des extraits aqueux, alcoolique et/ou hydro-alcooliques de partie de la plante Theobroma cacao.

10. Utilisation selon la revendication 9, **caractérisé en ce que** l'extrait végétal mimétique β-endorphine est un extrait hydro-alcoolique de coque de fève de cacao.

11. Procédé de traitement cosmétique pour améliorer la fonction barrière et/ou l'hydratation et/ou la résistance à la pollution de la peau et/ou des muqueuses **caractérisé en ce que** l'on applique une quantité efficace d'au moins un agent choisi parmi la β-endorphine, ses fragments et/ou les mimétiques de l'activité de la β-endorphine sur les kératinocytes de la peau.

12. Procédé selon la revendication 11 **caractérisé en ce que** l'agent est appliqué dans une composition pour application topique comprenant en outre au moins un composé choisi parmi les agents hydratants; les agents dépigmentants; les agents anti-glycation; les inhibiteurs de NO-synthase; les agents stimulant la synthèse de macromolécules dermiques ou épidermiques et/ou empêchant leur dégradation; les agents stimulant la prolifération des fibroblastes et/ou des kératinocytes ou stimulant la différenciation des kératinocytes; les agents myorelaxants; les agents tenseurs; les agents anti-pollution et/ou anti-radicalaire, les filtres solaires, et leurs mélanges.
